(19) Europäisches Patentamt
European Patent Office
Office européen des brevets

(11) EP 3 604 346 A1

(12) EUROPEAN PATENT APPLICATION

(43) Date of publication:
**05.02.2020 Bulletin 2020/06**

(21) Application number: **18186618.7**

(22) Date of filing: **31.07.2018**

(51) Int Cl.:
***C08B 3/08*** (2006.01) ***C08B 3/10*** (2006.01)
***C08B 3/14*** (2006.01) ***C08B 3/22*** (2006.01)
***C08B 31/02*** (2006.01) ***C08B 37/08*** (2006.01)
***C08L 1/10*** (2006.01) ***C08B 15/00*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Procter & Gamble International Operations SA**
**1213 Geneva (CH)**

(72) Inventors:
- **FERNANDEZ-PRIETO, Susana**
**1853 Strombeek-Bever (BE)**
- **SMETS, Johan**
**1853 Strombeek-Bever (BE)**
- **DE BORGGRAEVE, Wim Michel**
**B-3001 HEVERLEE (BE)**
- **PIRAS, Carmen Cristina**
**B-3001 HEVERLEE (BE)**

(74) Representative: **P&G Patent Belgium UK**
**N.V. Procter & Gamble Services Company S.A.**
**Temselaan 100**
**1853 Strombeek-Bever (BE)**

# (54) PROCESS FOR MAKING A CONSUMER PRODUCT COMPRISING MODIFIED POLYSACCHARIDES

(57) A process for preparing a consumer product comprising a chemically modified polysaccharide, the process comprising the steps of combining a slurry comprising polysaccharide with a reactant to form a polysaccharide-reactant mixture, wherein the reactant comprises an ester group; combining a base with the polysaccharide-reactant mixture to form a polysaccharide-reactant-base mixture; and allowing the polysaccharide-reactant-base mixture to form a transesterified polysaccharide mixture, wherein the transesterified polysaccharide mixture comprises an alcohol.

EP 3 604 346 A1

## Description

FIELD OF THE INVENTION

A process for making a consumer product comprising a modified polysaccharide is disclosed. In particular, a process which inserts chemical functionalities to polysaccharides whilst retaining the morphology of unmodified polysaccharides at a reduced need for organic solvent during the chemical modification process.

BACKGROUND OF THE INVENTION

Polysaccharides, such as cellulose, are renewable, biodegradable materials that provide a range of benefits to a wide range of applications. Such applications include coatings, packaging material, and fabric and home care consumer goods. Polysaccharides are known to improve for example the dry time, sprayability, viscosity and pigment dispersion in coatings (K. J. Edgar, C. M. Buchanan, J. S. Debenham, P. A. Rundquist, B. D. Seiler, M. C. Shelton and D. Tindall, Prog Polym Sci, 2001, 26, 1605-1688; M. Edge, N. S. Allen, T. S. Jewitt and C. V. Horie, Polym Degrad Stabil, 1989, 26, 221-229). Polysaccharides such as microfibrillated cellulose are known to improve the rheology profile in fabric care applications (US 7,749,332, EP0596550). Chemical modifications of such polysaccharides can provide further benefits such as modification of the hydrophobicity, modification of the rheology properties of the polysaccharides, or improved compatibility with other ingredients when formulated into consumer products.

Unfortunately, traditional chemical modifications of polysaccharides require the introduction of organic solvents or ionic liquids during the chemical modification step. Organic solvents (such as toluene) and ionic liquids (such as 1-allyl-3-methylimidazolium chloride) are typically not biodegradable and/or renewable and hence require after the introduction an additional removal step during the modification process which increase process complexity and hence cost.

Alternative polysaccharide modifications include ball milling which allows avoiding the use of organic solvents. However, the disadvantage is that some of the desired properties of the unmodified polysaccharide, such as the ability to efficiently structure liquid compositions, are negatively affected upon ball milling as the morphology of the polysaccharide is altered. Furthermore, as temperature increases due to the friction, ball milling can even lead to thermal degradation of the polysaccharide.

Hence, a need remains for a process to chemically modify polysaccharides whilst retaining the morphology of the unmodified polysaccharides without the need to intentionally add high levels of organic solvents or ionic liquids during the transesterification step. A further need is to minimize process complexity to reduce cost.

US 2772266 A relates to the preparation of cellulose acetate in which the cellulose is esterified in 2 steps, in the first of which the cellulose is substantially completely esterified, and in the second of which sulfuric acid is added to prevent gelation of the cellulose acetate in the esterification mass. US 2024381 A relates to the production of simple and mixed esters of cellulose, more especially cellulose acetates.

SUMMARY OF THE INVENTION

The present invention relates to a process for making a consumer product comprising a modified polysaccharide. The process comprises the modification of polysaccharides comprising the steps of providing a slurry comprising an undissolved polysaccharide and a liquid carrier, combining the polysaccharide slurry with a reactant and a base followed by a transesterification process. The resulting product is added to an unfinished consumer product or part thereof to make a finished consumer product. The process of modifying chitosan of the present invention exhibits good modification yield whilst retaining the morphology of the unmodified polysaccharide without the need for addition of ionic liquids or high level of organic solvents during the transesterification process.

The present invention also relates to consumer products comprising modified polysaccharides obtained by the same process.

The present invention also relates to an intermediate composition comprising a polysaccharide, a reactant, and a base.

The present invention further relates to the use of transesterified polysaccharide in consumer products, the transesterified polysaccharide obtainable according to the process according to the present invention to provide dynamic yield stress to a liquid consumer product.

DETAILED DESCRIPTION OF THE INVENTION

Definitions

As used herein, articles such as "a" and "an" when used in a claim, are understood to mean one or more of

what is claimed or described.

As used herein, the terms "include", "includes" and "including" are meant to be non-limiting.

By "consumer product" is herein meant a product intended to be used or consumed in the form in which it is sold. Preferably, the consumer product is a liquid or a solid which has been made by processing a liquid even if not all the ingredients of the product were processed in liquid form.

The consumer product can be baby care, beauty care excluding sun care, fabric and home care, family care, feminine care, snack and/or beverage products intended to be used or consumed in the form in which it is sold, and is not intended for subsequent commercial manufacture or modification. Such products include but are not limited to diapers, bibs, wipes; products for treating hair (human, dog, and/or cat), including bleaching, coloring, dyeing, conditioning, shampooing, styling; deodorants and antiperspirants; personal cleansing; cosmetics; skin care including application of creams, lotions, and other topically applied products for consumer use; and shaving products, products for treating fabrics, hard surfaces and any other surfaces in the area of fabric and home care, including: air care, car care, dishwashing, fabric conditioning (including softening), laundry detergency, laundry and rinse additive and/or care, hard surface cleaning and/or treatment, and other cleaning for consumer or institutional use; products relating to bath tissue, facial tissue, paper handkerchiefs, and/or paper towels; tampons, feminine napkins; products relating to oral care including toothpastes, tooth gels, tooth rinses, denture adhesives, tooth whitening; pet health and nutrition, and water purification; processed food products intended primarily for consumption between customary meals or as a meal accompaniment (non-limiting examples include potato chips, tortilla chips, popcorn, pretzels, corn chips, cereal bars, vegetable chips or crisps, snack mixes, party mixes, multigrain chips, snack crackers, cheese snacks, pork rinds, corn snacks, pellet snacks, extruded snacks and bagel chips); and coffee.

Preferably, the consumer products are cleaning or care products.

Unless otherwise noted, all component or composition levels are in reference to the active portion of that component or composition, and are exclusive of impurities, for example, residual solvents or by-products, which may be present in commercially available sources of such components or compositions.

All percentages and ratios are calculated by weight unless otherwise indicated. All percentages and ratios are calculated based on the total composition unless otherwise indicated.

All measurements are performed at 25°C unless otherwise specified.

As used herein, all boiling and freezing points are measured at atmospheric pressure.

Process for preparing a chemically modified polysaccharide

The process for preparing a chemically modified polysaccharide according to the present invention comprises the steps:

a) making a slurry comprising an undissolved polysaccharide and a liquid carrier;
b) combining said slurry with a reactant to form a polysaccharide-reactant mixture, wherein the reactant comprises an ester group;
c) combining a base with the polysaccharide-reactant mixture to form a polysaccharide-reactant-base mixture; and
d) allowing the polysaccharide-reactant-base mixture to form a transesterified polysaccharide mixture, wherein the transesterified polysaccharide mixture comprises an alcohol.

In preferred processes, there is no intentional addition of an organic solvent, an ionic liquid, or mixtures thereof; preferably wherein the levels of organic solvent and/or ionic liquid, if present, in any of the steps c to d is less than 10 %, preferably below 5%, even more preferably less than 0.1%.

Slurry comprising polysaccharide

With the term "slurry" we herein mean a composition comprising an undissolved polysaccharide into a liquid carrier. Preferably, the liquid carrier is an aqueous solution. Preferably the aqueous solution comprises a bactericide to control bacterial growth within the slurry.

The level of undissolved polysaccharide is preferably from 0.1 to 40%, more preferably from 0.5 to 20%, even more preferably from 0.5 to 10% by weight of the polysaccharide slurry. Higher levels of undissolved polysaccharide lead to higher viscosities and hence more difficult handling of the slurry.

Preferably, the polysaccharide comprises monomers selected from the group consisting of glucose, fructose, xylose, mannose, galactose, rhamnose, arabinose, and mixtures thereof, preferably the monomers are condensed through glycosidic bonds, more preferably the monomers are condensed through beta-1,4-glycosidic bonds or alpha-1,3-glycosidic bonds; even more preferably the polysaccharide is cellulose; most preferably, the polysaccharide is cellulose fiber. With cellulose fibers we herein mean microfibrillated cellulose and nanocellulose. Cellulose fibers provide

efficient structuring properties when formulated into liquid compositions; e.g. liquid consumer products.

The cellulose fibers can be of bacterial or botanical origin, i.e. produced by fermentation or extracted from vegetables, plants, fruits or wood. Cellulose fiber sources may be selected from the group consisting of citrus peels, such as lemons, oranges and/or grapefruit; fruits, such as apples, bananas and/or pear; vegetables such as carrots, peas, potatoes and/or chicory; plants such as bamboo, jute, abaca, flax, cotton and/or sisal, cereals, and different wood sources such as spruces, eucalyptus and/or oak. Preferably, the cellulose fiber source is selected from the group consisting of wood or plants, in particular, spruce, eucalyptus, jute and sisal.

The content of cellulose in the cellulose fibers will vary depending on the source and treatment applied for the extraction of the fibers, and will typically range from 15% to 100%, preferably above 30%, more preferably above 50%, and even more preferably above 80% of cellulose by weight of the cellulose fibers.

Such cellulose fibers may comprise pectin, hemicellulose, proteins, lignin and other impurities inherent to the cellulose based material source such as ash, metals, salts and combinations thereof. The cellulose fibers are preferably substantially non-ionic. Such fibers are commercially available, for instance Citri-Fi 100FG from Fiberstar, Herbacel® Classic from Herbafood, and Exilva® from Borregaard.

The cellulose fibers may have an average diameter from 10 nm to 350 nm, preferably from 30 nm to 250 nm, more preferably from 50 nm to 200 nm.

In one aspect when the liquid carrier of the slurry is an aqueous composition, prior to combining the undissolved polysaccharide with reactant, the slurry is washed with a solvent, preferably wherein the solvent is ethanol or propane-1,2-diol, even more preferably wherein the solvent is ethanol.

Polysaccharide-reactant mixture

According to step b) of the present invention, a slurry comprising polysaccharide is combined with a reactant to form a polysaccharide-reactant mixture. The reactant is essential to provide a chemical functionality to the polysaccharide. The reactant of the present invention comprises an ester group which is essential to enable transesterification in the process according to the present invention.

Preferably, the boiling point of the reactant is above 80°C, more preferably above 100°C, even more preferably above 120°C, to reduce evaporation of the reactant during the reaction.

Non-limiting examples of preferred reactants with their CAS number, chemical structure, and boiling point are listed in Table 1. More preferably, the reactant is selected from the list consisting of acetoacetate alkyl ester, methyl butyrate, ethyl valerate, ethyl-2-aminobenzoate, and mixtures thereof; most preferably the reactant is an acetoacetate alkyl.

Table 1: preferred reactants with their CAS number chemical structure and boiling point.

| Reactant name | CAS number | Chemical structure | Boiling point |
|---|---|---|---|
| Ethyl acetoacetate | 141-97-9 | 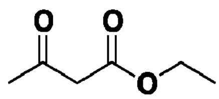 | 181 °C |
| Ethyl-2-methylacetoacetate | 609-14-3 | 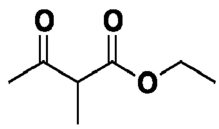 | 187 °C |
| Ethyl-2-chloroacetoacetate | 609-15-4 | 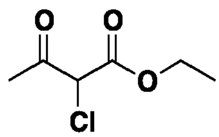 | 107 °C |
| Ethyl-4-chloroacetoacetate | 638-07-3 | 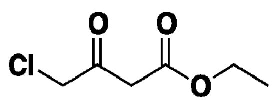 | 115 °C |
| Methyl-4-methoxy acetoacetate | 66762-68-3 | 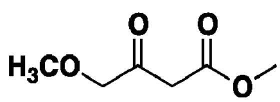 | 89 °C |

(continued)

| Reactant name | CAS number | Chemical structure | Boiling point |
|---|---|---|---|
| Methyl butyrate | 623-42-7 | 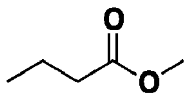 | 102-103 °C |
| Ethyl-3-hydroxybutyrate | 5405-41-4 | 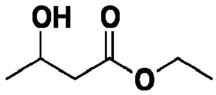 | 170 °C |
| Ethyl valerate | 539-82-2 | 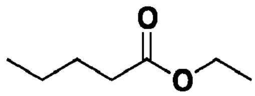 | 144-145 °C |
| Ethyl-5-bromovalerate | 14660-52-7 | 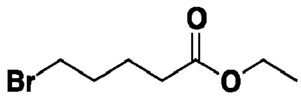 | 104-109 °C |
| Ethyl octanoate | 106-32-1 | 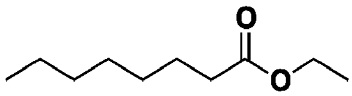 | 206-208 °C |
| Ethyl benzoate | 93-89-0 | 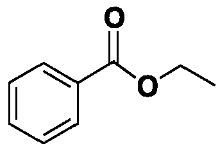 | 212 °C |
| Ethyl-2-aminobenzoate | 87-25-2 | 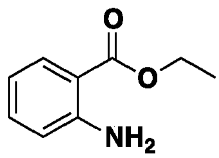 | 129-130 °C |
| Ethyl phenylacetate | 101-97-3 | 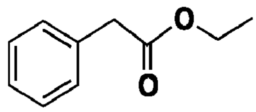 | 229 °C |
| Ethyl-3-phenylpropionate | 2021-28-5 | 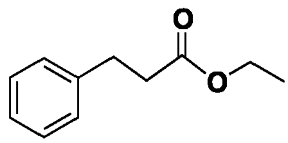 | 247-248 °C |

To further improve the efficiency of the transesterification process of step d), the level of aqueous carrier in the polysaccharide-reactant mixture is less than 10%, preferably less than 5%, more preferably less than 3%, even more preferably less than 1%. It is believed that the presence of water hinders the transesterification process.

Polysaccharide-reactant-base mixture

In step c) according to the present invention, a base is brought in contact with the polysaccharide-reactant mixture to form a polysaccharide-reactant-base mixture.

In preferred polysaccharide-reactant mixtures the water level is less than 10%, preferably less than 5%, more preferably less than 3%, even more preferably less than 1%. A low water level in the polysaccharide-reactant mixture further improves the transesterification process.

The base is used to catalyse the transesterification reaction between the reactant and the polysaccharide within the polysaccharide-reactant-base mixture. Suitable bases include alkaline metal alkoxides, alkaline metal hydroxides,

and non-ionic organic bases. Preferred bases are carbonates because of their low toxicity and low corrosivity. In addition, carbonates are easy to use and have the advantage to form bicarbonate instead of water as a by-product and hence help to avoiding ester hydrolysis. Especially preferred bases are $Na_2CO_3$, $Cs_2CO_3$, $K_2CO_3$, and mixtures thereof. Most preferably, the base catalyst is $Na_2CO_3$ and/or $Cs_2CO_3$.

In one aspect of the invention the polysaccharide-reactant-base mixture is an intermediate mixture wherein the reactant comprises an ester; preferably the reactant is selected from the group consisting of ethyl acetoacetate, ethyl-2-methylacetoacetate, ethyl-2-chloroacetoacetate, ethyl-4-chloroacetoacetate, methyl-4-methoxy acetoacetate, methyl butyrate, ethyl-3-hydroxybutyrate, ethyl valerate, ethyl-5-bromovalerate, ethyl octanoate, ethyl benzoate, ethyl-2-aminobenzoate, ethyl phenylacetate, ethyl-3-phenylpropionate, and mixtures thereof; most preferably the reactant is an acetoacetate alkyl ester; wherein the base is selected from the group consisting of metal alkoxides, alkaline metal hydroxide, organic bases, and mixtures thereof, preferably the base is selected from the group consisting of $K_2CO_3$, $CsCO_3$, $Na_2CO_3$, and mixtures thereof; wherein the level of organic solvent and/or ionic liquid is less than 10%, preferably less than 5%, more preferably less than 0.1%.

Transesterification

The chemical modification of the polysaccharide according to the present invention in step d) is a transesterification process. The transesterification occurs in the polysaccharide-reactant-base mixture wherein the reactant reacts with the polysaccharide to form a transesterified polysaccharide mixture wherein the transesterified polysaccharide mixture comprises an alcohol as a result of the transesterification process.

In a one aspect, the invention comprises a further step of removing the alcohol, preferably removing the alcohol under vacuum to obtain a transesterified polysaccharide mixture wherein the level of alcohol by weight of the mixture is less than 10%, preferably less than 5%, even more preferably less than 1%.

In case the boiling point of the reactant is below the reaction temperature, a reflux system is required to avoid loss of the reactant. In a preferred process, the polysaccharide-reactant-base mixture is heated to a temperature above the freezing point of the reactant and below the boiling point of the reactant, preferably at least 10°C below the boiling point of the reactant, more preferably at least 20°C below the boiling point of the reactant, most preferably 30°C below the boiling point of the reactant to improve the transesterification process.

In a further preferred process, the transesterification process lasts from 30 minutes to 120 minutes, preferably from 40 minutes to 100 minutes, most preferably from 50 minutes to 80 minutes.

The percentage of functionalization influences the compatibility of the modified polysaccharide with other ingredients when formulated into a consumer product. However, when the percentage of functionalization is high, the rheological properties of the modified polysaccharide are reduced. Preferably, the chemically modified polysaccharide has a percentage of functionalization of more than 0.5% and less than 50%. More preferably the percentage of functionalization is more than 0.5% and less than 30%, even more preferably more than 0.5% and less than 20%, most preferably more than 0.5% and less than 10%. With percentage of functionalization we herein mean the degree of substitution and is determined as described in the Methods.

In one aspect of the invention, the chemically modified polysaccharide has a formula

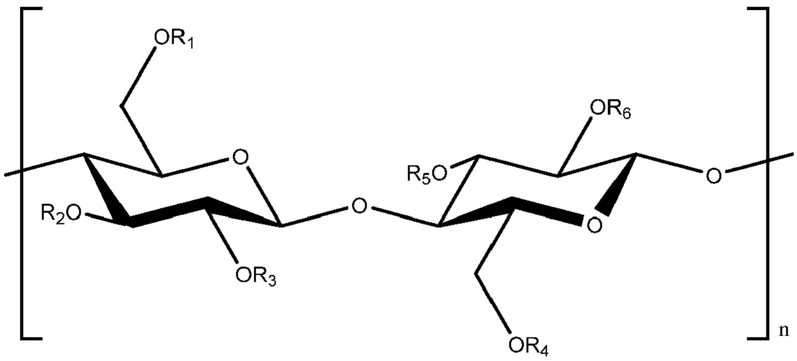

wherein $R_1$, $R_2$, $R_3$, $R_4$, $R_5$, and $R_6$, are independently selected from hydrogen or from the group consisting of

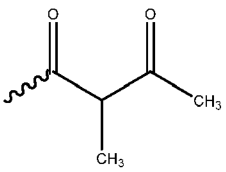 , 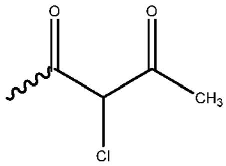 , 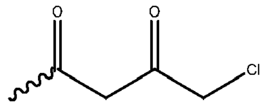 , 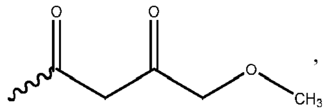 ,

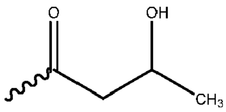 , 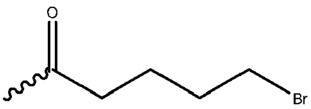 , 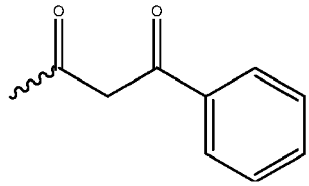 ,

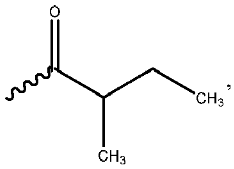 , 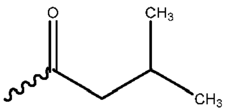 , 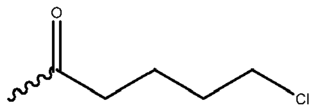

;

wherein at least one of $R_1$, $R_2$, $R_3$, $R_4$, $R_5$, and $R_6$ is not a hydrogen; wherein n is from 200 to 3000, preferably from 300 to 2800, more preferably from 500 to 2500. The wavy line represents where the R group links to the polysaccharide structure.

Cross-linking

Optionally, the process further comprises a cross-linking step wherein the transesterified polysaccharide is cross-linked preferably with chitosan or L-lysine. The optional additional cross-linking further improves the compatibility with other ingredients when formulated into a liquid composition and still have a dynamic yield stress. The Applicants believe that the improved compatibility is a result of the cationic charge provided by chitosan and L-lysine.

Once the modified polysaccharide is obtained, it is added to an un-finised consumer product or part thereof to give rise to a finished consumer product. The product is preferably a cleaning or a care product.

Use of an esterified polysaccharide

The invention further relates to the use of an esterified polysaccharide obtainable according to the process of the present invention in a liquid composition, preferably a fabric care composition, to provide yield stress and improved compatibility with other ingredients of the composition.

METHODS

IR spectroscopy

Instrument details: Attenuated total reflection (ATR) Fourier transformation infrared spectroscopy (FTIR) was used to determine the main functional groups of synthesized derivatives. All the spectra are obtained by an Alpha Bruker Spectrometer apparatus in a wavenumber range of 4,000-550 $cm^{-1}$.

Sample preparation: approximately 0.65 grams of transesterified polysaccharide slurry were weighed in a glass vial (Alemania Glas GmBH, 26.5 mm diameter). The samples were placed in the oven (Memmert GmBH) at 150 °C to remove the water until a dry solid residue was obtained. The resulting solid residue (approximately 20 mg) was placed onto the sample holder of the equipment without any further treatment.

Result: The formation of the transesterified polysaccharide was verified by IR spectroscopy. The IR spectra of the synthesized transesterified polysaccharide displays the characteristic band of the ester bond in the region 1,720-1,750 $cm^{-1}$.

Degree of substitution (DS) and % of functionalisation

The degree of substitution of each transesterified polysaccharide was determined by titration. The titration was performed in triplicate.

Determination of dry matter: After the transesterification reaction, about 2 g of each aqueous transesterified polysaccharide slurry was weighed in a vial (Alemania Glas GmbH, 26.5 mm diameter). The samples were dried in the oven (Memmert GmbH) at 150 °C until complete removal of water. The samples were then weighed again to calculate the dry matter, which was obtained as a percentage. Each measurement was performed three times.

The dry matter is calculated using the following equation:

$$\text{Dry matter } \% = (\text{weight of dry sample x } 100) / (\text{weight of the aqueous transesterified polysaccharide slurry})$$

Sample preparation and titration: each sample (about 3 g) is dispersed as an aqueous slurry in a solution of aqueous ethanol (70 %, Ethanol Absolut - Fisher and Milli-Q water) in a 20 mL of total volume of solvent (including the water content of the slurry) in a 100 mL round bottom flask. The resulting suspension was stirred for 30 minutes using a magnetic stirrer (Yellow Line - MST Basic C) at 20 °C at a 600 rpm rotational speed. After 30 minutes, sodium hydroxide 0.5 M (20 mL, Sigma Aldrich) was added. The reaction was performed for 48 or 72 hours under mixing at 600 rpm at 20 °C. The round bottom flask is closed with a rubber top and sealed with parafilm to prevent solvent evaporation. After this time, the excess of sodium hydroxide is back titrated with hydrochloric acid 0.5 M (Sigma Aldrich) in the presence of phenolphthalein (Sigma Aldrich - 5 drops) as an indicator. The addition of hydrochloric acid is stopped when a colour change from pink to transparent was observed, therefore the volume of hydrochloric acid added varied each time depending on the % of functionalisation. A blank measurement was performed each time.

Blank measurement: sodium hydroxide 0.5 M (20 mL) was stirred in a solution of aqueous ethanol (70 % - 20 mL) in a 100 mL round bottom flask for 48 or 72 hours. After this time, sodium hydroxide was titrated with hydrochloric acid 0.5 M in the presence of phenolphthalein (5 drops) as an indicator. The addition of hydrochloric acid was stopped when a colour change from pink to transparent was observed.

Degree of substitution (DS): The degree of substitution is obtained from the following equation:

$$DS = M.W._{monomer} \text{ x } [(\text{vol } HCl_{blank} \text{ x conc}) - (\text{vol } HCl_{titration} \text{ x conc})] / w - \{[(\text{vol } HCl_{blank} \text{ x conc}) - (\text{vol } HCl_{titration} \text{ x conc})] \text{ x } M.W._{grafted\ ester\ functionality}\}$$

wherein

$M.W._{monomer}$ = molecular weight of monomer unit
Vol = volume (L)
Conc = concentration of hydrochloric acid (M)
$M.W._{grafted\ ester\ functionality}$ = molecular weight of the grafted ester functionality
w = weight of the solid content in the aqueous slurry.

Percentage of functionalisation: The percentage of functionalisation is obtained from the following equation:

$$\%\text{ functionalisation} = DS \text{ x } 100 / \text{number of -OH groups present in the monomer}$$

Water determination via Karl Fisher

Water percentage in the reaction media wss determined via Karl Fisher titration using a Mettler DL 31, Metrohm 795 Volumetric Karl Fischer Titrator or equivalent in drift control mode using a two-component volumetric reagents. First, a blank was titrated, then 0.5 gram sample were added into the titrator filled with HYDRANALTM Solvent (Honeywell) and titrated with HYDRANALTM Titrant 5. The instrument provided the % of water in the sample already taking into account the water in the blank.

Dynamic yield stress

Sample preparation for dynamic yield stress measurement: transesterified polysaccharide aqueous slurry was added to a surfactant model matrix and mixed for 5 minutes at 21.000 rpm using a ultra-turrax with S25N-10G dispersing element. Final composition was:

| Ingredient | wt % |
|---|---|
| Linear Alkylbenzene Sulphonic Acid (HLAS) | 10 |
| transesterified polysaccharide (as dry material) | 0.25 |
| Monoethanolamine | to pH 8 |
| Water | to 100% |

Dynamic yield stress was measured using a controlled stress rheometer (such as an HAAKE MARS from Thermo Scientific, or equivalent), using a 60 mm 1° cone-plate and a gap size of 0.052 microns at 20°C. The dynamic yield stress was obtained by measuring quasi steady state shear stress as a function of shear rate starting from 10 $s^{-1}$ to $10^{-4}$ $s^{-1}$, taking 25 points logarithmically distributed over the shear rate range. Quasi-steady state is defined as the shear stress value once variation of shear stress over time is less than 3%, after at least 30 seconds and a maximum of 60 seconds at a given shear rate. Variation of shear stress over time was continuously evaluated by comparison of the average shear stress measured over periods of 3 seconds. If after 60 seconds measurement at a certain shear rate, the shear stress value varies more than 3%, the final shear stress measurement was defined as the quasi state value for calculation purposes. Shear stress data was then fitted using least squares method in logarithmic space as a function of shear rate following a Herschel - Bulkley model:

$$\tau = \tau_0 + k\dot{\gamma}^n$$

wherein $\tau$ is the measured equilibrium quasi steady state shear stress at each applied shear rate $\dot{\gamma}$, $\tau_0$ is the fitted dynamic yield stress. *k* and *n* are fitting parameters.

EXAMPLES

The undissolved polyssacharide used for examples 1-16 was microfibrillated cellulose (MFC) provided by Borregaard as a slurry of undissolved microfibrillated cellulose in an aqueous carrier (Exilva Forte, level of 3% microfibrillated cellulose by weight of the slurry) and was used as supplied. All the other commercial reagents and solvents were purchased from Sigma-Aldrich, VWR, Fisher and Alfa Aesar.

Equivalent to 1 gram of dry polysaccharide, the polysaccharide aqueous slurry was weighed in a 250 mL beaker, diluted with 100 mL of demineralized water and stirred for 5 mins at 600 rpm with a magnetic stirrer (Yellow Line - MST Basic C). The slurry was filtered on a sintered glass funnel by vacuum filtration using a Vacuubrand GmBH + CO pump. The filtrate was mixed with 50 mL ethanol Absolut (Fisher) on the filter and filtered again under vacuum. This last step was repeated twice. The resulting filtrate was transferred into a 100 mL beaker and stirred with 50 mL ethanol for 5 minutes on the magnetic stirrer. Then, the slurry was filtered again on a sintered glass funnel by vacuum filtration. This step was performed twice. The resulting filtrate with a water level of less than 10% was transferred into a 100 mL beaker and stirred with 50 mL of the selected reactant to form a microfibrillated cellulose-reactant mixture.

The microfibrillated cellulose-reactant mixture was stirred at a 600 rpm with the magnetic stirrer. The mixture was heated to 105 - 110°C using an oil bath and the base (such as $Na_2CO_3$, $Cs_2CO_3$ supplied from Acros Organics) was added to form a microfibrillated cellulose-reactant-base mixture.

The microfibrillated cellulose-reactant-base mixture was allowed to transesterify at this temperature. The reaction time was varied to vary the degree of substitution.

Once the transesterification reaction was considered completed, stirring was stopped and the beaker was removed from the oil bath. 50 mL Ethanol were added and the transesterified polysaccharide was collected by vacuum filtration. The transesterified polysaccharide is then mixed on the filter with 50 mL ethanol and filtered again under vacuum. The resulting transesterified polysaccharide filtrate was subsequently transferred into a 100 mL beaker and stirred with 50 mL ethanol for 5 mins with the magnetic stirrer. The transesterified polysaccharide filtrate was collected by vacuum filtration. This same procedure was performed twice with ethanol (2 x 50 mL) and it is repeated for three times using water as dispersing agent (3 x 50 mL). The final product was homogenized using an Ultra-Turrax homogenizer

(IKA) for 5 mins at 21,000 rpm. The formation of the ester bond and the absence of starting material were confirmed by IR spectroscopy. 33.33 grams MFC slurry were used in each example.

Table 2: Detailed composition of examples 1-16. In the examples with an asterisk, a reflux system was applied the since reaction temperature was above the boiling point of the reactant. The reaction time in example 3 was 2 hrs while in the other examples 1-2, 4-16 the reaction time was 1 hour.

| Ex. | Reactant | Base | Base addition [mg] | Reaction temperature [°C] | % functionalization [%] |
|---|---|---|---|---|---|
| 1 | Ethyl acetoacetate | CS2CO3 | 60.31 | 105°C | 15.7% |
| 2 | Ethyl acetoacetate | CS2CO3 | 40.20 | 105°C | 3.33% |
| 3 | Ethyl acetoacetate | CS2CO3 | 40.20 | 105°C | 3.67% |
| 4 | Ethyl acetoacetate | $Na_2CO_3$ | 29.43 | 105°C | 17% |
| 5 | Ethyl acetoacetate | $Na_2CO_3$ | 19.62 | 105°C | 7.33% |
| 6 | Ethyl acetoacetate | $Na_2CO_3$ | 13.08 | 105°C | 1% |
| 7 | Ethyl-2-methy lacetoacetate | $Na_2CO_3$ | 29.43 | 110 °C | 3.33% |
| 8* | Ethyl-2-chloroacetoacetate | $Na_2CO_3$ | 29.43 | 110 °C | 3.67% |
| 9* | Ethyl-4-chloroacetoacetate | $Na_2CO_3$ | 29.43 | 110 °C | 10.33% |
| 10* | Methyl-4-methoxy acetoacetate | $Na_2CO_3$ | 29.43 | 110 °C | 4% |
| 11* | Methyl butyrate | $Na_2CO_3$ | 29.43 | 110 °C | 2.9% |
| 12 | Ethyl-3-hydroxybutyrate | $Na_2CO_3$ | 29.43 | 110 °C | 13.67% |
| 13 | Ethyl valerate | $Na_2CO_3$ | 29.43 | 110 °C | 33.33% |
| 14 | Ethyl benzoate | $Na_2CO_3$ | 29.43 | 110 °C | 5.33% |
| 15 | Ethyl-2-aminobenzoate | $Na_2CO_3$ | 29.43 | 110 °C | 13.33% |
| 16 | Ethyl phenylacetate | $Na_2CO_3$ | 29.43 | 110 °C | 17.67% |

Examples 1 to 16 demonstrates that the process according to the present invention results in transesterification. In addition, examples 1-6 illustrate the effect of base, base level, and reaction temperature on the percentage of functionalization. To better control the level of functionalization % we did not remove the alcohol formed in the above examples.

Example 17: 0.402 gram of chitosan (molecular weight 200000g/mol, Glentham Life Sciences LTD) were dissolved in 40 mL of a 1% aqueous solution of acetic acid and a gel was instantly formed. 0.5 grams of transesterified polysaccharide from example 1 was added. The mixture was diluted with 60 mL water to facilitate mixing. The reaction was stirred for 3 hours at 20°C and product formation was monitored every hour by IR spectroscopy. After 3 hours, the reaction was stopped and the product was purified by multiple washing with water (4 x 50 mL). The final chitosan cross-linked transesterified polysaccharide was homogenized using an Ultra-Turrax for 5 mins at 21000 rpm. The formation of the enamine bond was confirmed by IR spectroscopy.

Example 18: 0.451 grams L(+)-Lysine monohydrochloride (99%, Acros Organics NV) was dissolved in 25mL water. 0.5 grams of transesterified polysaccharide from example 1 was added and the composition was stirred for 24 hours at 20°C. The obtained product was purified by multiple washing with water (4 x 50 mL). The final L-lysine cross-linked transesterified polysaccharide was homogenized using an Ultra-Turrax for 5 mins at 21000 rpm. The formation of the enamine bond was confirmed by IR spectroscopy.

Example A (comparative): transesterification using ball milling

Initially, 1 gram of wood derived cellulose fibers pulped and bleached without being defibrillated (10% activity) was added to each of the two zirconium oxide chambers (Planetary Micro Mill, PULVERISETTE 7 premium line from Fritsch). Each chamber (80 mL volume) was filled with 25 zirconium oxide milling balls (10 mm diameter). The process was carried out at 400 and 800rpm rotational speed and between 2 and 10 minutes. After 2 minutes, there was no change in the cellulose fibers. However, when increasing the milling time, fibers were burnt. Since suitable conditions without burning the fibers could not be identified, no reactant was added.

Without being bound by theory, it is believed that burning of fibers was due to the bleaching. Therefore, raw jute fibers (no treatment) were used instead and 1 gram of dried jute fibers was added to each of the two zirconium oxide chambers (Planetary Micro Mill, PULVERISETTE 7 premium line from Fritsch) together with 0.14g ethyl acetoacetate and 0.020 equivalents of sodium carbonate. Each chamber (80 mL volume) was filled with 25 zirconium oxide milling balls (10 mm diameter). The process was carried out at 800 rpm rotational speed for 2 minutes. The percentage of functionalization obtained was 5%. However, because the size of the fibers was modified, they have no rheology modification properties. Modified fibers precipitate at the bottom of the bottle not being possible to perform rheology characterization.

Table 3: The dynamic yield stress of the different examples as described in the Methods section.

| Example | Dynamic Yield Stress (Pa) |
|---|---|
| 1 | 0.29 |
| 11 | 0.22 |
| 12 | 0.14 |
| 13 | 0.21 |
| 15 | 0.23 |
| 17 | 0.04 |
| 18 | 0.15 |
| A (Comparative) | <0.001 |

No yield stress was measured with the chemically modified polysaccharide of comparative example A even though the % of functionalization was similar to example 1-6. It is believed that the lack of a dynamic yield stress with comparative example A was caused because the size of the fibers was modified during the milling. In addition, the chemically modified fibers of comparative example A precipitated at the bottom of the recipient when added to the surfactant model matrix (see Methods).

From example 1, 11-18 it is clear that a dynamic yield stress was still obtained after the chemical modification according to the present invention when the chemically modified polysaccharide was formulated into the model surfactant matrix (see Methods). Furthermore, it is clear that L-lysine cross-linking (ex. 18) resulted in a higher dynamic yield stress than cross-linking with chitosan (ex. 17).

## Claims

**1.** A process for making a consumer product comprising a chemically modified polysaccharide, the process comprising the steps of:

a) providing a polysaccharide slurry comprising an undissolved polysaccharide and a liquid carrier;
b) combining the polysaccharide slurry with a reactant to form a polysaccharide-reactant mixture, wherein the reactant comprises an ester group;
c) combining a base with the polysaccharide-reactant mixture to form a polysaccharide-reactant-base mixture;
d) allowing the polysaccharide-reactant-base mixture to form a transesterified polysaccharide mixture, wherein the formed transesterified polysaccharide mixture comprises an alcohol;
e) adding the mixture resulting from step d) to an unfinished consumer product or part thereof to make a consumer product.

**2.** The process according to Claim 1 wherein in step d) the polysaccharide-reactant-base mixture is heated to a temperature above the freezing point of the reactant and below the boiling point of the reactant, preferably at least 10°C below the boiling point of the reactant, more preferably at least 20°C below the boiling point of the reactant, most preferably 30°C below the boiling point of the reactant.

**3.** The process according to any preceding claim wherein step d) lasts from 30 minutes to 120 minutes, preferably from 40 minutes to 100 minutes, most preferably from 50 minutes to 80 minutes.

**4.** The process according to any preceding claim wherein the polysaccharide comprises monomers selected from the group consisting of glucose, fructose, xylose, mannose, galactose, rhamnose, arabinose, and mixtures thereof, preferably the monomers are condensed through glycosidic bonds, more preferably the monomers are condensed through beta 1,4-glycosidic bonds or alpha-1,3-glycosidic bonds; even more preferably the polysaccharide is cellulose, most preferably microfibrillated cellulose.

**5.** The process according to any preceding claim wherein the base is selected from the group consisting of metal alkoxides, alkaline metal hydroxide, organic bases, preferably the base is a carbonate, more preferably the base is selected from the group consisting of $K_2CO_3$, $CsCO_3$, $Na_2CO_3$, and mixtures thereof, most preferably the base is selected from $CsCO_3$ and $Na_2CO_3$, and mixtures thereof.

**6.** The process according to any preceding claim wherein the reactant is selected from the group consisting of acetoacetate alkyl ester, methyl butyrate, ethyl-3-hydroxybutyrate, ethyl valerate, ethyl-5-bromovalerate, ethyl octanoate, ethyl benzoate, ethyl-2-aminobenzoate, ethyl phenylacetate, ethyl-3-phenylpropionate, and mixtures thereof; preferably the reactant is selected from the list consisting of acetoacetate alkyl ester, methyl butyrate, ethyl valerate, ethyl-2-aminobenzoate, and mixtures thereof; most preferably the reactant is an acetoacetate alkyl ester.

**7.** The process according to any preceding claim wherein the water level of the polysaccharide-reactant mixture is less than 10%, preferably less than 5%, more preferably less than 3%, even more preferably less than 1%.

**8.** The process according to any preceding claim wherein there is no intentional addition of an organic solvent, an ionic liquid, or mixtures thereof, in any of the steps a) to d), preferably wherein the levels of organic solvent and/or ionic liquid, if present, in any of the steps c and/or d is less than 10 %, preferably below 5%, even more preferably less than 0.1%.

**9.** The process according to any preceding claim wherein the chemically modified polysaccharide has a percentage of functionalization of more than 0.5% and less than 50%, preferably less than 30%, more preferably less than 20%, most preferably less than 10%.

**10.** The process according to any preceding claim further comprises the step:
removing the alcohol, preferably removing the alcohol under vacuum to obtain a transesterified polysaccharide mixture wherein the level of alcohol by weight of the mixture is less than 10%, preferably less than 5%, even more preferably less than 1%.

**11.** The process according to any preceding claim further comprises the step of cross-linking the transesterified polysaccharide, preferably the transesterified polysaccharide is cross-linked with chitosan or L-lysine.

**12.** The process according to any preceding claim wherein the consumer product is a cleaning or care product.

**13.** An intermediate composition comprising a polysaccharide, a reactant, and a base wherein

a) the reactant comprises an ester, preferably the reactant is selected from the group consisting of ethyl acetoacetate, ethyl-2-methylacetoacetate, ethyl-2-chloroacetoacetate, ethyl-4-chloroacetoacetate, methyl-4-methoxy acetoacetate, methyl butyrate, ethyl-3-hydroxybutyrate, ethyl valerate, ethyl-5-bromovalerate, ethyl octanoate, ethyl benzoate, ethyl-2-aminobenzoate, ethyl phenylacetate, ethyl-3-phenylpropionate, and mixtures thereof; most preferably the reactant is an acetoacetate alkyl ester;
b) the base is selected from the group consisting of metal alkoxides, alkaline metal hydroxide, organic bases, and mixtures thereof, preferably the base is selected from the group consisting of $K_2CO_3$, $CsCO_3$, $Na_2CO_3$, and mixtures thereof;
c) the level of organic solvent and/or ionic liquid is less than 10%, preferably less than 5%, more preferably less

than 0.1%.

**14.** A consumer product comprising a chemically modified polysaccharide having formula

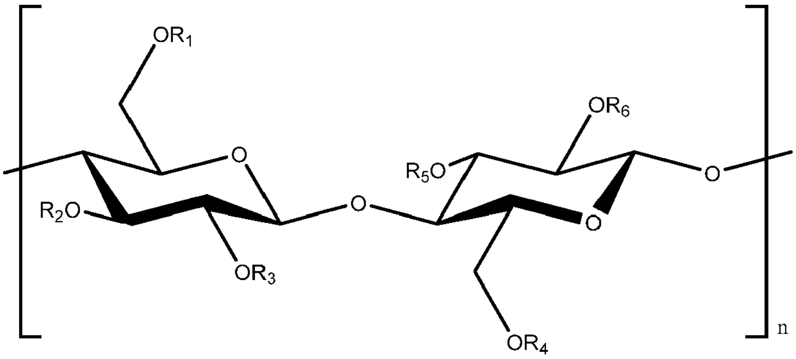

wherein $R_1$, $R_2$, $R_3$, $R_4$, $R_5$, and $R_6$, are independently selected from hydrogen or from the group consisting of

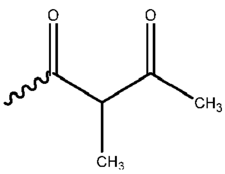 , 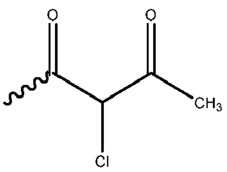 , 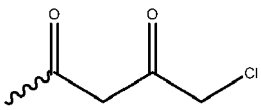 , 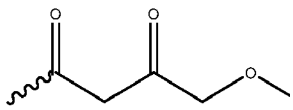

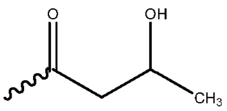 , 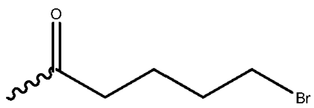 , 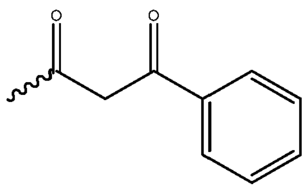 ,

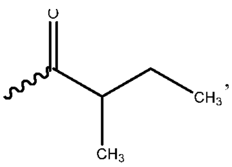 , 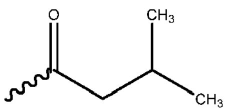 , 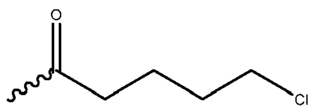

;

wherein at least one of $R_1$, $R_2$, $R_3$, $R_4$, $R_5$, and $R_6$ is not a hydrogen; wherein n is from 200 to 3000, preferably from 300 to 2800, more preferably from 500 to 2500.

**15.** Use of transesterified polysaccharide obtainable according to the process steps a) to d) of any one of claims 1 to 12 to provide dynamic yield stress to a liquid consumer product.

Europäisches Patentamt
European Patent Office
Office européen des brevets

# EUROPEAN SEARCH REPORT

Application Number
EP 18 18 6618

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CA 838 117 A (NAT STARCH CHEM CORP) 31 March 1970 (1970-03-31) * page 2, lines 1-21 * * page 3, lines 8-18 * * page 6, lines 11-27 * * example 1 * ----- | 1-5,8,9, 11-13 | INV. C08B3/08 C08B3/10 C08B3/14 C08B3/22 C08B31/02 C08B37/08 C08L1/10 C08B15/00 |
| X | JP 2010 254798 A (KAO CORP) 11 November 2010 (2010-11-11) * abstract * * paragraph [0028] * * claims 1-2 * * example 1 * ----- | 1-15 | |
| X | US 2007/142804 A1 (BERNARD BOBBY L [US]) 21 June 2007 (2007-06-21) * paragraphs [0001] - [0002], [0006] - [0010], [0016] - [0017] * ----- | 14 | |
| X | EP 3 339 408 A1 (PROCTER & GAMBLE [US]) 27 June 2018 (2018-06-27) * paragraphs [0006], [0014] - [0017] * * paragraphs [0117] - [0121]; examples 5-8; table 1 * ----- | 15 | TECHNICAL FIELDS SEARCHED (IPC) C08B C08L |

The present search report has been drawn up for all claims

1

| Place of search | Date of completion of the search | Examiner |
|---|---|---|
| Munich | 28 January 2019 | Lartigue, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 18 18 6618

This annex lists the patent family members relating to the patent documents cited in the above-mentioned European search report.
The members are as contained in the European Patent Office EDP file on
The European Patent Office is in no way liable for these particulars which are merely given for the purpose of information.

28-01-2019

| Patent document cited in search report | Publication date | Patent family member(s) | Publication date |
|---|---|---|---|
| CA 838117 A | 31-03-1970 | NONE | |
| JP 2010254798 A | 11-11-2010 | NONE | |
| US 2007142804 A1 | 21-06-2007 | BR PI0619232 A2 | 06-09-2016 |
| | | CN 101331250 A | 24-12-2008 |
| | | EP 1960572 A2 | 27-08-2008 |
| | | US 2007142804 A1 | 21-06-2007 |
| | | WO 2007078554 A2 | 12-07-2007 |
| EP 3339408 A1 | 27-06-2018 | EP 3339408 A1 | 27-06-2018 |
| | | US 2018179472 A1 | 28-06-2018 |
| | | WO 2018118447 A1 | 28-06-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7749332 B
- EP 0596550 A
- US 2772266 A
- US 2024381 A


### Non-patent literature cited in the description


- **K. J. EDGAR ; C. M. BUCHANAN ; J. S. DEBENHAM ; P. A. RUNDQUIST ; B. D. SEILER ; M. C. SHELTON ; D. TINDALL.** *Prog Polym Sci,* 2001, vol. 26, 1605-1688
- **M. EDGE ; N. S. ALLEN ; T. S. JEWITT ; C. V. HORIE.** *Polym Degrad Stabil,* 1989, vol. 26, 221-229